(19)

Europäisches
Patentamt

European
Patent Office

Office européen
des brevets

(11) **EP 4 292 589 A1**

(12) **EUROPEAN PATENT APPLICATION**
published in accordance with Art. 153(4) EPC

(43) Date of publication:
**20.12.2023 Bulletin 2023/51**

(21) Application number: **21903591.2**

(22) Date of filing: **07.10.2021**

(51) International Patent Classification (IPC):
*A61K 9/51* (2006.01)          *A61K 39/00* (2006.01)
*A61K 41/00* (2020.01)        *A61P 35/00* (2006.01)

(52) Cooperative Patent Classification (CPC):
**A61K 9/51; A61K 39/00; A61K 41/00; A61P 35/00**

(86) International application number:
**PCT/KR2021/013746**

(87) International publication number:
**WO 2022/124544 (16.06.2022 Gazette 2022/24)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **07.12.2020 KR 20200169289
09.08.2021 KR 20210104313**

(71) Applicant: **Fortuga Bio
Seoul 04790 (KR)**

(72) Inventors:
• **HONG, Jinkee**
  **Seoul 03722 (KR)**
• **HA, Sangjun**
  **Seoul 03722 (KR)**
• **CHOI, Daheui**
  **Seoul 03722 (KR)**
• **KANG, Taegun**
  **Seoul 03722 (KR)**
• **KIM, Taihyun**
  **Seoul 03722 (KR)**

(74) Representative: **J A Kemp LLP
80 Turnmill Street
London EC1M 5QU (GB)**

(54) **DENDRITIC CELL-MIMICKED NANOSTRUCTURE FOR APPLICATION TO CANCER IMMUNOTHERAPY, AND FABRICATION METHOD THEREFOR**

(57)    The present invention relates to a dendritic cell-mimicked nanostructure and a fabrication method therefor and, more specifically, to a nanostructure in which a shell including a cell membrane of dendritic cell-derived lipid molecules is introduced to a nanoparticle core in order to take advantage of the surface antigen-presenting ability of dendritic cells and which enables targeting without disappearance in vivo, thereby providing an effect of inducing an effective immune response.

**EP 4 292 589 A1**

**Figure 1**

**Description**

[Technical Field]

The present invention relates to a nanostructure that mimics immune cells using dendritic cells and a fabrication method thereof.

[Background Art]

Cancer ranks first among the causes of death in Korea. Representative cancer treatment methods include surgery to remove the cancer, radiation therapy, and chemical therapy, but these treatment methods have problems of poor prognosis and serious side effects.

The main cause of cancer development may include tumor formation of cancer cells that are not eliminated by an immune response due to decreased immunity or immune evasion of cancer cells. Accordingly, cancer immunotherapy, which may help treat cancer by increasing the patient's own immune response, may be a fundamental cancer therapy method. Currently developed cancer immunotherapies mostly include direct injection of drugs that enhance immune functions, but drug injection still has limitations in that the delivery efficiency is very low and additional side effects may exist. Accordingly, research is focusing on the development of therapies that may dramatically increase cancer treatment efficiency, reduce recurrence rate and side effects, and increase immune functions.

Meanwhile, research on nano-bio convergence technology that combines nanotechnology with the biomedical field has continued. However, most nanomaterials are obtained through synthesis, and surface modification using nanomaterials involves a process of adding a composite, and thus, side effects may occur, such as in-vivo toxicity, induction of unwanted immune responses, and induction of cancer. Therefore, side effects may be minimized by using a surface modification method that directly mimics the living body, and various functions may be implemented according to biological characteristics, thereby overcoming the limitations of existing nanotechnology.

In particular, nanoparticle-cellularization technology is a technology that physically cloaks the surface of nanoparticles by using the entire cell membrane of a specific cell as a coating material, and may maintain the complex properties of cell membranes along with proteins, lipids, and carbohydrates to implement characteristics of a specific cell as they are on the surface of nanoparticles.

Based on the technology that introduced platelets to the surface of poly(lacticco-glycolic)acid (PLGA) particles for the first time in 2015 by Liangfang Zhang's research team at the University of California, the nanoparticle-cellular technology has been applied to various cells up to date.

The study was reported in which an immunocompatible nanocarrier was prepared by loading doxorubicin on a PLGA core and then coating the PLGA core with a red blood cell membrane to remove solid tumors or nanoparticles with hybrid cell membranes were prepared by simultaneously coating a red blood cell membrane and a cancer cell membrane with melanin nanoparticles to increase in-vivo circulation time and enhance tumor targeting ability.

However, most of conventional cell membrane coating technologies were focused on cancer cells and blood cells, and its application was also mainly focused on studies aimed at stable delivery, etc. in the blood. In addition, when a cancer cell membrane is directly introduced as an antigen and delivered into the body, there are disadvantages, such as decreased immune response due to antigen resistance, resistance to cancer cell-derived substances, and the like. Accordingly, it is very necessary to develop immunotherapy agents capable of having the functions of direct antigen-presenting immune cells to induce differentiation and proliferation of T cells without intermediate processes.

[Prior Arts]

Non-Patent Document

Hu, Che-Ming J., et al. Nature, 2015, 526 (7571) 118-12
Brian T. Luk et al., Theranostics. 2016, 6(7), 1004-101
Q. Jiang et al., Biomaterials 2019, 192, 292-308.

[Disclosure]

[Technical Problem]

The present invention is to achieve a cancer treatment effect by increasing the immune response by using cells with an antigen-presenting function beyond material limitations of the prior art. Specifically, the present invention is to provide excellent immunotherapy effects by producing a nanostructure that mimics dendritic cells to directly activate cytotoxic T cells according to an antigen-presenting function of dendritic cells in vivo, thereby inducing selective death of cancer cells and increasing the immune responses. In addition, an object of the present invention is to provide much enhanced cancer immunotherapy effects by using targeting and photothermal effects of nanoparticles.

Another object of the present invention is to provide a method for fabricating a dendritic cell-mimicked nanostructure that is thermodynamically very stable and is optimized to promote proliferation and differentiation of T cells while staying in the body for a long period of time.

[Technical Solution]

In order to achieve the object, the present invention provides a dendritic cell-mimicked nanostructure using nanoparticle-cellularization technology. Specifically, the present invention provides a nanostructure including a nanoparticle core; and a shell including a cell membrane of lipid molecules derived from dendritic cells, in which the shell includes a double layer of lipid molecules.

In an aspect of the present invention, the shell may be 5 to 20 nm.

In an aspect of the present invention, the surface coverage of the nanostructure may be 70% or higher, preferably 85% or higher.

In an aspect of the present invention, the absolute value of the surface potential of the nanostructure may be smaller than the absolute value of the surface potential of lipid molecules derived from dendritic cells.

In an aspect of the present invention, the nanostructure may be fabricated by fusing the nanoparticles and liposomes prepared by sonicating the dendritic cell-derived cell membrane.

In an aspect of the present invention, the nanostructure may be fabricated by co-extruding the liposomes and the nanoparticles.

In an aspect of the present invention, the particle size of the liposome may be 200 nm or less.

In an aspect of the present invention, the surface zeta potential of the nanostructure may be - 35 to - 25 mV.

Another aspect of the present invention provides a fabrication method of a dendritic cell-mimicked nanostructure, and specifically including: purifying a cell membrane from dendritic cells; forming a cell membrane suspension by sonicating the cell membrane; obtaining liposomes by filtering the cell membrane suspension through a membrane filter; and mixing nanoparticles and the liposomes and then obtaining nanoparticles into which the cell membrane has been introduced by filter extrusion.

In an aspect of the present invention, the nanoparticles and the liposomes may be mixed in a volume ratio of 1 : 150 to 1 : 300.

In an aspect of the present invention, the fabrication method may further include pulsing an antigen to the nanoparticles into which the cell membrane has been introduced.

In an aspect of the present invention, the antigen may be a peptide or protein derived from a tumor antigen.

In an aspect of the present invention, the average particle size of the liposome may be 200 nm or less.

In an aspect of the present invention, the nanoparticles into which the cell membrane is introduced may have a smaller absolute value of surface potential than the nanoparticles before the cell membrane introduction.

Yet another aspect of the present invention provides a cancer immunotherapy including the dendritic cell-mimicked nanostructure described above.

Yet another aspect of the present invention provides a pharmaceutical composition for treating cancer including the dendritic cell-mimicked nanostructure described above.

Yet another aspect of the present invention provides a dendritic cell-mimicked nanostructure fabricated by the fabrication method described above.

[Advantageous Effects]

According to the present invention, a dendritic cell-mimicked nanostructure may provide a cancer immunotherapy effect with enhanced functions by introducing the antigen-presenting ability of dendritic cells into the nanoparticle surface to additionally imparting a targeting function and a photothermal effect function of nanoparticles without disappearing while preserving the antigen-presenting function of dendritic cells.

According to the present invention, the dendritic cell-mimicked nanostructure mimics immune cells using dendritic cells, and stays in the body for a long period of time and continuously induces the proliferation and differentiation of antigen-specific T cells by introducing a dendritic cell-derived cell membrane into a shell, thereby providing an effect of increasing the immune responses.

According to the present invention, the dendritic cell-mimicked nanostructure may minimize side effects by using a patient's own immune system and selectively remove micro-sized cancer cells or metastasized cancer cells that are difficult to diagnose, as compared to existing chemotherapy or radiation therapy-based anticancer treatment methods.

According to the present invention, the dendritic cell-mimicked nanostructure itself has anticancer activity to be used as a cancer immunotherapy, and may expect stronger anticancer activity through combined use with other anticancer drugs, so as to be useful in developing new anticancer drugs that minimize side effects and exhibit strong anticancer effects.

[Description of Drawings]

FIG. 1A is a schematic diagram illustrating a process of fabricating a dendritic cell-mimicked nanostructure by filter extruding activated dendritic cells and gold nanoparticles through nanoparticle-cellularization technology. FIG. 1B is a schematic diagram illustrating that the dendritic cell-mimicked nanostructure according to the present invention increases immune responses through binding to T cells.

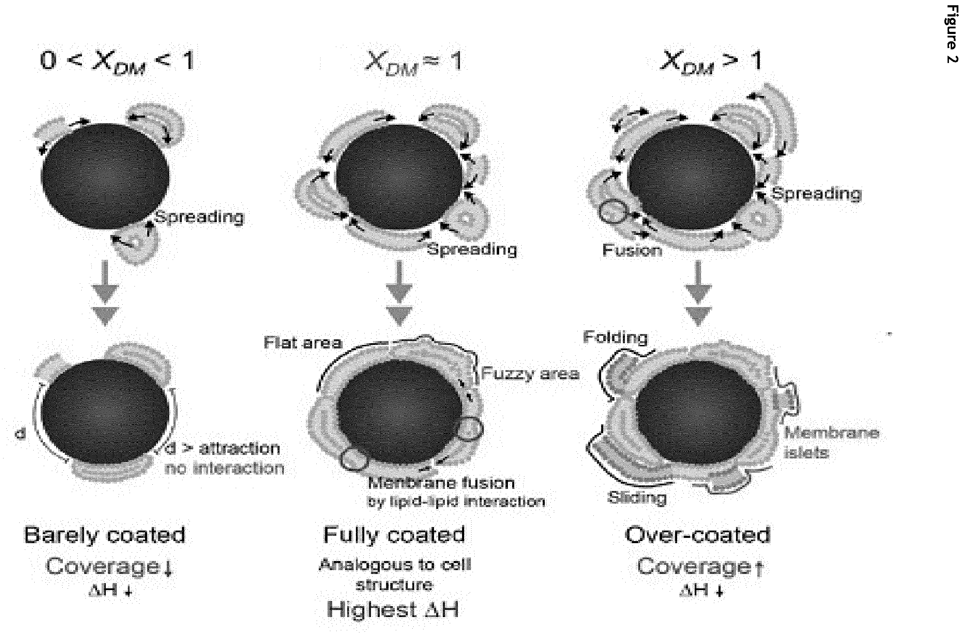

FIG. 2 is a schematic diagram illustrating the degree of surface coating according to a mixing ratio of nanoparticles and liposomes of the present invention.

FIG. 3 shows SEM images and surface coverages of dendritic cell-mimicked nanostructures fabricated according to Example 1 of the present invention and Comparative Example 1.

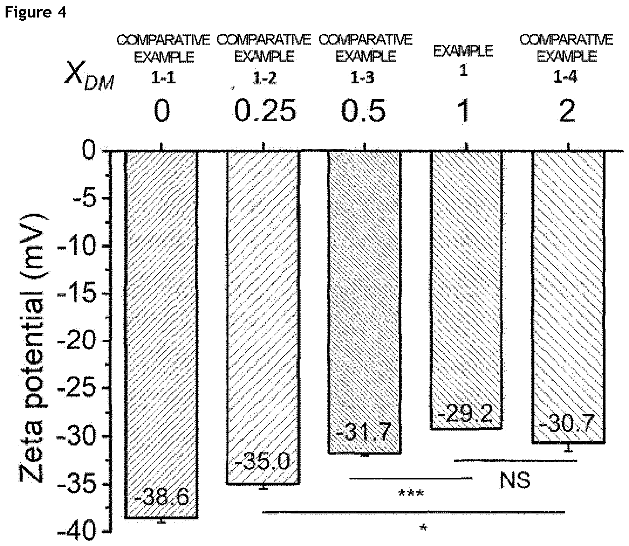

FIG. 4 is a graph showing surface zeta potentials of dendritic cell-mimicked nanostructures fabricated according to Example 1 of the present invention and Comparative Example 1.

FIG. 5 is a graph showing expression rates of CTV, CD44, IFNg, TNF$\alpha$, and IL-2, as a result of co-culturing antigen-specific T cells with the dendritic cell-mimicked nanostructure according to Experimental Example 1 of the present invention.

FIG. 6 is a graph showing a melting temperature of the dendritic cell-mimicked nanostructure according to Experimental Example 1 of the present invention.

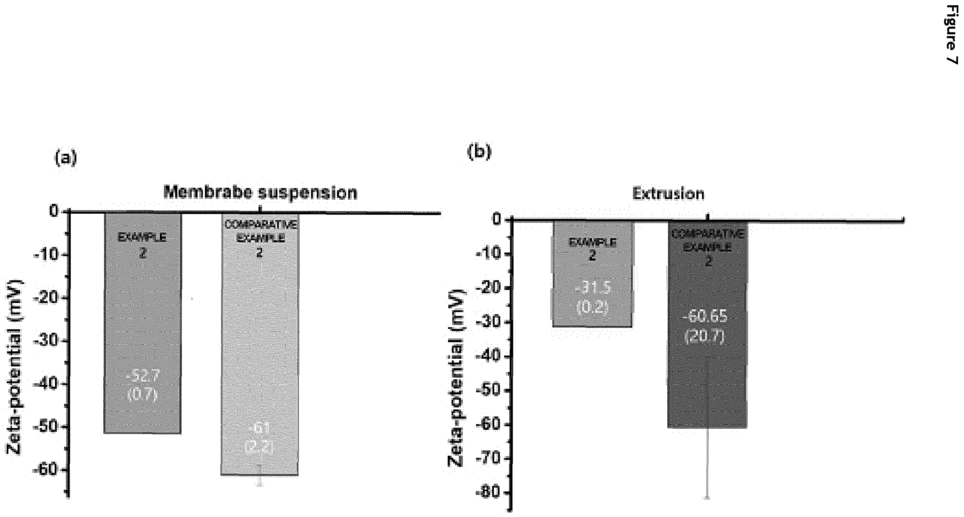

FIG. 7 illustrates surface zeta potentials before (Membrane suspension) and after (Extrusion) co-extrusion of the dendritic cell-mimicked nanostructure fabricated without sonication according to Comparative Example 2 of the present invention.

FIG. 8 shows electron microscopic images of gold nanoparticles (NPs), an extracted dendritic cell membrane (DCm), a dendritic cell-mimicked nanostructure (DCm-NP), and a dendritic cell-mimicked nanostructure (DCm-NP/Ag (Antigen)) pulsed with an antigen according to Experimental Example 3 of the present invention.

FIG. 9 is a graph showing surface zeta potential values of gold nanoparticles (Gold NP), an extracted dendritic cell membrane (DCm), a dendritic cell-mimicked nanostructure (DCm-NP), and a dendritic cell-mimicked nanostructure (DCm-NP/Ag) pulsed with an antigen according to Experimental Example 3 of the present invention.

FIG. 10 illustrates data of confirming the presence or absence of CD80, CD86, MHC class I, and II, which are major surface proteins of dendritic cells, on the surface of the dendritic cell-mimicked nanostructure according to Experimental Example 3 of the present invention through FACS.

FIGS. 11A and 11B illustrate T cell proliferation and differentiation efficiency through an in vitro experiment in a group of dendritic cell-mimicked nanostructure (DCm-NP) and bone marrow-derived dendritic cells without pulsing each antigen, a group of pulsing an OVA$_{257-264}$ antigen, and a group of pulsing a GP$_{33-41}$ antigen according to Experimental Example 3-5 of the present invention.

FIGS. 12A and 12B illustrate T cell proliferation and differentiation efficiency through an In vivo experiment in a group of dendritic cell-mimicked nanostructure (DCm-NP) and bone marrow-derived dendritic cells without pulsing each antigen, and a group of pulsing a GP$_{33-41}$ antigen according to Experimental Example 3-6 of the present invention.

FIG. 13 illustrates changes in T cell proliferation and differentiation efficiency over 10, 20, and 30 days, as a stability test result of the dendritic cell-mimicked nanostructure (DCm-NP) according to Experimental Example 3-7 of the present invention.

[Best Mode for the Invention]

Hereinafter, the present invention will be described in detail.

Unless otherwise defined herein, all technical and scientific terms have meanings commonly understood by those skilled in the art to which the present invention pertains, and the terms used in the description of the present invention are merely to effectively describe specific embodiments and are not intended to limit the present invention.

The embodiments and accompanying drawings of this specification are intended to easily understand and implement the present invention by those skilled in the art, and contents that may obscure the gist of the present invention may be omitted from the embodiments and drawings, and the present invention is not limited to the embodiments and drawings. The present invention may be embodied in other forms within the range without changing the technical idea of the present invention.

Further, the description of known effects and configurations that may make the gist of the present invention unnecessarily ambiguous will be omitted in the following description.

In addition, in describing the components of the present invention, terms including first, second, A, B, (a), (b), and the like may be used. These terms are just intended to distinguish the components from other components, and the terms do not limit the nature, sequence, or order of the components.

In addition, as used in the specification of the present invention, the singular forms may be intended to include plural forms, unless the context clearly dictates otherwise.

Hereinafter, a dendritic cell-mimicked nanostructure according to the present invention, a fabrication method thereof, a dendritic cell-mimicked nanostructure fabricated according to the fabrication method thereof, a cancer immunotherapy including the same, and a pharmaceutical composition for treating cancer will be described in detail.

The present invention provides a dendritic cell-mimicked nanostructure using nanoparticle-cellularization technology. The antigen-presenting ability of dendritic cells is introduced onto the surface of nanoparticles, thereby (1) preserving the antigen-presenting function of dendritic cells and simultaneously (2) exhibiting a significantly enhanced cancer immunotherapy effect through targeting function and photothermal effect of nanoparticles.

Nanoparticle-cellularization technology proceeds in three major steps: (1) extracting the cell membrane of living cells without damage, (2) generating nano liposomes of a certain size using a filter extruder, and then (3) repeating filter extrusion with nanoparticles to be coated to transfer the entire cell membrane to the nanoparticle surface by applying physical force. In addition, there are a variety of technologies derived from the fact that nanoparticles that mimic specific cells may be fabricated as desired regardless of a type of cell membrane or nanoparticle.

In the case of a cancer therapy vaccine using dendritic cells, a strategy was used to activate cancer antigen-specific T cells in cancer patients by expressing cancer antigens in dendritic cells differentiated from monocytes in the patient's blood then injecting the dendritic cells back into the cancer patient. However, this type of dendritic cell cancer therapy vaccine did not show a clear anticancer effect due to limitations such as the short survival period, weak T cell stimulation ability, and the like of the injected dendritic cells.

Further, in addition to cancer immunotherapy, there have been efforts to use dendritic cells with induced immune tolerance as a therapy for treatment of autoimmune diseases including rheumatoid arthritis, but similarly, the immunetolerant dendritic cells did not show sufficient therapeutic effect due to the short survival period, weak immunosuppressive ability, and the like.

The present invention is intended to provide a new type of immunotherapy to overcome these limitations, and unlike conventional technologies, the immunotherapy has a long survival period in vivo, ensures structural stability even during long-term storage, and may exhibit a significantly improved effect even on T cell activation ability.

The present invention relates to a nanostructure including a nanoparticle core; and a shell including a cell membrane of lipid molecules derived from dendritic cells, in which the shell includes a double layer of lipid molecules.

The nanoparticles have biocompatibility, and preferably have a property of absorbing light in the near-infrared region and generating heat. Specifically, for example, the nanoparticles may be metal nanoparticles, organic polymer nanoparticles, melanin nanoparticles, graphene nanoparticles, inorganic nanoparticles, or a combination thereof that may exhibit a photothermal effect, but are not necessarily limited thereto. However, in a preferred embodiment of the present invention, the nanoparticles may be gold nanoparticles that have a high reduction potential to maintain a safe state in the body and to be easily surface modified.

Dendritic cells (DCs) are powerful antigen presenting cells (APCs) and play an important role in inducing immune responses and regulating immunity in vivo. The DC may induce a primary immune response by activating naive T cells that have never encountered an antigen, and function as an immune cell that may induce antigen-specific acquired memory immunity.

In addition, not only major histocompatibility complex I/II (MHC I/II) present on the cell membrane surface, but also co-stimulatory molecules such as CD80 and CD86 and cell adhesion molecules such as ICAM-1 are highly expressed, and exhibit a strong antigen presentation function by secreting large amounts of various cytokines such as interferon, IL-12, and IL-18, which are related to T cell activation.

Specifically, a series of processes in which dendritic cells differentiate T cells in vivo and activate immune responses to remove antigenic substances are as follows.

First, the dendritic cells exist in peripheral tissues, and receive activation signals from external stimuli to be matured. At the same time, foreign protein antigens are presented in peptide form to MHC class I and II. Thereafter, the dendritic cell migrates to the draining lymphnode and binds to a T cell having a T cell receptor (TCR) capable of binding to an MHC-peptide complex on the surface of the dendritic cell, and co-stimulatory ligands such as CD80 and CD86 expressed on the dendritic cell binds to CD28, a co-stimulatory receptor expressed on the T cell to completely activate T cells. The activated T cells proliferate and differentiate, migrate from lymph nodes to peripheral tissues, and eliminate cells (e.g. cancer cells) expressing foreign antigens.

In the present invention, in order to implement the antigen presenting function of dendritic cells, a dendritic cell-derived cell membrane is introduced onto the surface of nanoparticles. A cell membrane of dendritic cell-derived lipid molecules includes a double layer of lipid molecules, and at this time, a first lipid molecule layer facing the outside of the shell is preferably oriented to exhibit an electrically stronger negative charge than a second lipid molecule layer facing the nanoparticle surface. In the fabrication method of the dendritic cell-mimicked nanostructure according to the present invention to be described below, by sonicating the cell membrane of dendritic cell-derived lipid molecules, the first lipid molecule layer facing the outside of the shell may be oriented to exhibit an electrically stronger negative charge than the second lipid molecule layer facing the nanoparticle surface.

The dendritic cell-mimicked nanostructure according to the present invention is fabricated by extracting the cell membrane of activated dendritic cells and filter-extruding the cell membrane together with nanoparticles to induce the cell membrane to be introduced to the nanoparticle surface.

The shell including the cell membrane of the lipid molecules is preferably coated thinly and uniformly on the surface of the nanoparticle core in the range of 2 to 50 nm, preferably 5 to 20 nm. At this time, when the surface coverage is 70% or more, preferably 85% or more, and more preferably 90% or more, the shell is thermodynamically stable and may exhibit excellent stability of the nanostructure.

The dendritic cell-mimicked nanostructure according to the present invention is fabricated by fusing nanoparticles with liposomes prepared by sonicating the dendritic cell-derived cell membrane and the fusion may mean mixing and co-extruding liposomes and nanoparticles.

Specifically, the dendritic cell-derived cell membrane is sonicated and then filtered through a nano-sized membrane filter to obtain liposomes with a particle size of 200 nm or less. The particle size of the liposome may preferably be 50 to 150 nm.

The surface zeta potential of the dendritic cell-mimicked nanostructure according to the present invention may be - 40 to - 20 mV, specifically - 35 to - 25 mV.

Hereinafter, the fabrication method of the dendritic cell-mimicked nanostructure according to the present invention will be described in detail.

Specifically, the fabrication method includes (a) purifying the cell membrane from the dendritic cells; (b) forming a cell membrane suspension by sonicating the cell membrane; (c) filtering the cell membrane suspension through a membrane filter to obtain liposomes; and (d) mixing nanoparticles and the liposomes and then obtaining nanoparticles into which the cell membrane has been introduced by filter extrusion.

Furthermore, the present invention may further include pulsing an antigen to cell membrane-coated nanoparticles for antigen presentation to complete the nanostructure that functions as dendritic cells.

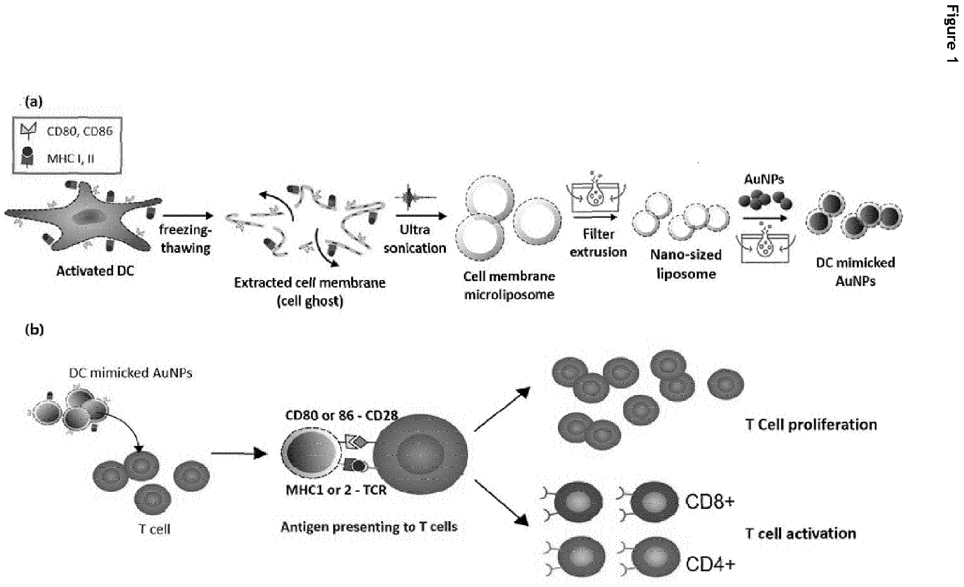

FIG. 1A illustrates a schematic diagram of fabricating a dendritic cell-mimicked nanostructure according to an example of the present invention. Bone marrow-derived dendritic cells may be extracted from mice to induce innate immune responses and adaptive immune responses. Specifically, when the cell membrane purified from the dendritic cells is mixed with predetermined nanoparticles in an appropriate ratio and filtered and extruded, the outer cell membrane, which has a relatively high negative zeta potential, and the negatively charged nanoparticles exhibit electrostatic repulsion. Accordingly, while a part with a relatively low negative zeta potential is adsorbed by interacting with the surface of the nanoparticles, the cell membrane is coated in a right-side-out orientation method, that is, in a desired direction of the cell membrane.

The dendritic cell-mimicked nanostructure according to the present invention may not only effectively increase the activity of T cells present in the body, but also stay in inflammation areas including tumors as well as lymph nodes due to its excellent permeability and long-term stable retention, and at this time, has an advantage of more effectively removing cancer cells and inflammatory cells due to the photothermal effect of nanoparticles by additional near-infrared ray irradiation to the area.

Specifically, the purifying of the cell membrane from the dendritic cells may be performed through rapid freezing-thawing and centrifugation. When the purified cell membrane is sonicated, the cell membrane suspension of hundreds of nanometers or several micrometers may be formed. Thereafter, the cell membrane suspension is filtered through a membrane filter to obtain cell membrane liposomes having desired sizes. Nanoparticles introduced with the cell membrane may be obtained by mixing the cell membrane liposomes and the nanoparticles and filter-extruding them. In other words, a form in which the cell membrane of the dendritic cell is coated on the surface of the nanoparticles is obtained. At this time, the coated surface coverage may be freely adjusted by varying the concentrations of nanoparticles and liposomes.

The mixing ratio of the nanoparticles and liposomes may be expressed by a value of Equation 1 below, which is represented as a ratio of the surface area of the cell membrane to the surface area of the nanoparticles. Specifically, for example, $X_{DM}$ in Equation 1 below may be 0.7 to 1.8. More preferably, $X_{DM}$ may be 0.9 to 1.3. In this case, most of the surface of the nanoparticles is coated with the cell membrane to represent a high surface coverage of 70% or higher or 85% or higher. However, if the mixing ratio has a value of $X_{DM} \fallingdotseq 2$, the surface of the nanoparticles may be overcoated, which may reduce thermodynamic stability.

[Equation 1]

$$\text{Mole fraction } (X_{DM}) = \frac{\text{Surface area of cell membrane}}{\text{Surface area of nanoparticles}}$$

According to Equation 1 above, the mixing ratio of nanoparticles and dendritic cell-derived liposomes may be determined depending on a type of nanoparticle, an average particle size, and a concentration of dendritic cells. At this time, since the dendritic cells have a radial shape developed with branch-like protrusions, it is preferred to calculate the surface area from the liposome shape.

According to the disclosure of the present invention, an optimal mixing ratio of nanoparticles and dendritic cell-derived liposomes may be calculated by comprehensively considering the concentrations of nanoparticles and liposomes and the surface area according to the type and size of nanoparticles.

Specifically, the optimal mixing ratio may be designed as described in Table 1 below.

[Table 1]

|  | Nanparticles | Liposomes |
|---|---|---|
| Number concentration ratio(A) | a | 1 |
| Unit surface area ratio (B) | 1 | b |
| Total surface area ratio (A x B) | a | b |
| Mixing ratio | b | 2a~3a |
| | b/3a~b/2a | 1 |

The number concentration ratio (A) may mean a ratio of the numbers of nanoparticles and liposomes contained per 1 ml. In other words, if there is one liposome in 1 ml, it means that A nanoparticle is contained in 1 ml. More precisely, the one liposome may be defined as $1 \times 10^6$.

A unit surface area ratio (B) is calculated based on the nanoparticles by calculating the surface area of each unit of nanoparticle and liposome. At this time, the nanoparticles and liposomes were considered as spherical shapes and calculated according to $4\pi r^2$ from the average particle diameters of the nanoparticles and liposomes. In addition, a total surface area ratio is a product value of the number concentration ratio (A) and the unit surface area ratio (B), and the total surface area ratio is a : b.

In the present invention, the nanoparticles may be sonicated to increase dispersion power before filter co-extrusion with cell membrane liposomes. In this case, it may be preferred to treat ultrasound to the extent that 2 to 3 nanoparticles may become unit particles. Accordingly, in order to implement ideal surface coverage in Equation 1 above, the mixing ratio of the sonicated unit nanoparticles and liposomes may be substantially b/3a : 1 to b/2a : 1.

The optimal mixing ratio of the nanoparticles and the dendritic cell-derived liposomes is able to implement a dendritic cell-mimicking nanostructure with 100% surface coverage so that the cell membrane may be coated on all nanoparticle surfaces according to Equation 1 above.

In the fabrication method of the dendritic cell-mimicked nanostructure according to the present invention, the pulsing of the antigen to the nanoparticles into which the cell membrane has been introduced is a step of loading the antigen onto the cell membrane by exposing the nanoparticles converted to the dendritic cells to the antigen, thereby inducing the activation of strong antigen-specific T cells.

The antigen may be a peptide or protein derived from a tumor antigen, and the tumor antigen may be a tumor-associated antigen or a tumor-specific antigen. Specifically, for example, in a mouse cancer model, the antigen may be a protein or peptide derived from ovalbumin (OVA), Lymphocytic choriomeningitis mammarenavirus (LCMV) glycoprotein, or retrovirus protein. More specifically, the antigen may be a cancer antigen peptide or protein of the $OVA_{257\text{-}264}$, $GP_{33\text{-}41}$, or p15E model.

In addition, human cancer antigens may be peptides or proteins derived from (1) tumor-associated antigens including HER2/Neu, tyrosinase, gp100, MART, HPV E6/E7, EBV EBNA-1, carcinoembryonic antigen, $\alpha$-fetoprotein, GM2, GD2, testis antigen, prostate antigen, and CD20, and (2) tumor-specific antigens, including a neoantigen that may be produced by various mutations. Preferably, the antigen may be one of the cancer antigen peptides described above or two or more different peptides.

The pulsing may be performed using various pulsing protocols known in the art, but more preferably, the pulsing may be performed by mixing and culturing with tumor antigen-derived peptides or proteins for 0.5 to 6 hours under humidified conditions of 5% $CO_2$ and 37°C.

Specifically, as a non-limiting example of the present invention, after treating $OVA_{257-264}$ and $GP_{33-41}$ antigens at a concentration of 0.1 to 0.3 μg/ml, and when treating the p15E antigen at a concentration of 2 to 7 μg/ml, the antigen may be pulsed by storing in an incubator at 37°C for 30 minutes.

The antigen-pulsed dendritic cell-mimicked nanostructure has an excellent cancer targeting function due to its nano size (negative targeting), is stable, and has a large surface area to its volume so as to be easily in contact with T cells. Accordingly, an effective anti-cancer immune response may be induced through activation of the dendritic cell-mimicked nanostructure and resulting activation of a specific T cell response. In addition, since there is no risk of death within the body, there is an advantage of having a very long circulation time in the body.

The fabricating process of the dendritic cell-mimicked nanostructure according to the present invention is simple, and has an advantage of being able to induce activation of T cells without an intermediate process by directly mimicking immune cells that have already undergone an immune response. In addition, when the photothermal effect of nanoparticles is added, the tumor suppressing effect is increased, so that the dendritic cell-mimicked nanostructure may be widely used as a cancer immunotherapy.

The present invention also provides a pharmaceutical composition for treating cancer including the dendritic cell-mimicked nanostructure.

The pharmaceutical composition of the present invention may further include a pharmaceutically acceptable carrier. The composition including the pharmaceutically acceptable carrier may be various oral or parenteral formulations, but is preferably a parenteral formulation. When the pharmaceutical composition is formulated, the formulation may be prepared by using diluents or excipients, such as a filler, an extender, a binder, a wetting agent, a disintegrating agent, and a surfactant, which are generally used. Solid formulations for oral administration include a tablet, a pill, a powder, a granule, a capsule, and the like, and the solid formulations may be prepared by mixing at least one excipient, for example, starch, calcium carbonate, sucrose or lactose, gelatin, and the like with at least one compound. Further, a lubricant such as magnesium stearate and talc may also be used in addition to the simple excipent. Liquid formulations for oral administration may correspond to a suspension, an oral liquid, an emulsion, a syrup, and the like, and may include various excipients, for example, a wetting agent, a sweetener, an aromatic agent, a preserving agent, and the like, in addition to water and liquid paraffin which are commonly used as simple diluents. Formulations for parenteral administration include a sterile aqueous solution, a non-aqueous solution, a suspension, an emulsion, a lyophilizing agent, and a suppository. As the non-aqueous solution and the suspension, propylene glycol, polyethylene glycol, vegetable oil such as olive oil, injectable ester such as ethyl oleate, and the like may be used. As a base of the suppository, witepsol, macrogol, tween 61, cacao butter, laurinum, glycerogelatin, and the like may be used.

The pharmaceutical composition may have any one formulation selected from the group consisting of tablets, pills, powders, granules, capsules, suspensions, liquids, emulsions, syrups, sterilized aqueous solutions, non-aqueous solvents, suspensions, emulsions, lyophilized agents, and suppositories. The pharmaceutical composition of the present invention may be administered orally or parenterally, and when administered parenterally, the pharmaceutical composition is able to be administered through various routes such as intravenous injection, intranasal inhalation, intramuscular administration, intraperitoneal administration, and transdermal absorption.

The pharmaceutical composition of the present invention is administered in a pharmaceutically effective dose. In the present invention, the term "pharmaceutically effective dose" refers to an amount enough to treat diseases at a reasonable benefit/risk ratio applicable to medical treatment, and an effective dose level may be determined according to factors including a kind of subject, the severity, age, gender, the activity of a drug, sensitivity to a drug, a time of administration, a route of administration, an excretion rate, duration of treatment, and drugs to be simultaneously used, and other factors well-known in the medical field.

The pharmaceutical composition of the present invention may be administered at a dose of 0.1 mg/kg to 1 g/kg, and more preferably at a dose of 1 mg/kg to 500 mg/kg. The dose may be appropriately adjusted according to the age, sex and condition of the patient.

The pharmaceutical composition of the present invention may be administered as an individual therapeutic agent or in combination with other anticancer agents, and sequentially or simultaneously administered with conventional anticancer agents. In addition, the pharmaceutical composition may be administered singly or in multiple. It is important to administer an amount capable of obtaining a maximum effect with a minimal amount without side effects in consideration with all the factors, and the amount thereof may be easily determined by those skilled in the art.

Furthermore, the present invention may provide a method for treating cancer in a subject, including administering the dendritic cell-mimicked nanostructure or a pharmaceutical composition including the dendritic cell-mimicked nanostructure to a subject from cancer. The pharmaceutical composition is administered to the subject to induce a tumor-specific immune response in the subject, or to treat and/or alleviate the symptoms of cancer in the subject. At this time, the subject may be a human or non-human mammal.

FIG. 1B shows an image of T cell stimulation of the fabricated dendritic cell-mimicked nanostructure according to the present invention. It is shown that the MHC class I, II/CD80, and CD86 of the nanostructure may bind to T cell receptor (TCR)/CD28 of T cells, respectively, to induce T cell proliferation and T cell differentiation.

Hereinafter, the present invention will be described in more detail below through Examples and Experimental Examples.

[Modes for the Invention]

### Example 1. Preparation of dendritic cell-mimicked nanostructure

Differentiated dendritic cells were extracted from bone marrow cells isolated from the femurs of 6- to 8-week-old Naive B6 mice (Orient Bio) and centrifuged at 2,000 rpm for 5 minutes to obtain pure dendritic cells. Thereafter, the cells were treated with a Protease Inhibitor Tablet-PBS buffer and dispersed at a concentration of 1 to $2 \times 10^6$ cells/ml. Then, only the cell membrane was purified through rapid freezing at -70°C, thawing at room temperature, and centrifugation. Thereafter, ultrasonic waves (VC505, Sonics & Materials) were applied at amplitude of 20% for a total of 60 cycles of 1 cycle/3 seconds with 3 seconds on/off and a 2-second cooling period between cycles to obtain a micro-scale cell membrane suspension, and the cell membrane suspension was filtered through a polycarbonate membrane with a nano-sized filter (pore sizes of 1 $\mu$m, 400 nm, 100 nm) to generate nano-liposomes with a diameter of each pore size. Considering the surface area of the cell membrane coated on the surface of the nanoparticles, it was assumed that $X_{DM}$ = 1 when the nanoparticles and liposomes had an optimized mixing ratio.

[Equation 1]

$$\text{Mole fraction } (X_{DM}) = \frac{\text{Surface area of cell membrane}}{\text{Surface area of nanoparticles}}$$

Accordingly, 5 $\mu$l of polystyrene (PS) nanoparticles and 1 ml of liposomes were mixed so that $X_{DM}$ = 1, and then filter-extruded together to prepare a dendritic cell-mimicked nanostructure. The prepared dendritic cell-mimicked nanostructure was treated with 0.2 $\mu$g/ml of GP-33 antigen at 37°C for about 30 minutes to induce the antigen to be presented on the surfaces of MHC class I and II and prepare the dendritic cell-mimicked nanostructure with the pulsed antigen.

### Example 2. Preparation of dendritic cell-mimicked nanostructure

A dendritic cell-mimicked nanostructure and an antigen-pulsed dendritic cell-mimicking nanostructure were prepared by the same method as in Example 1, except for using gold nanoparticles of 60 nm as the nanoparticles. The size of the dendritic cell-mimicked nanostructure was measured to be approximately 80 nm.

### Comparative Example 1. Preparation of dendritic cell-mimicked nanostructure by varying mixing ratio of nanoparticles and liposomes

A dendritic cell-mimicked nanostructure was prepared by performing the same method as in Example 1, except for mixing ratios shown in Table 2 below.

[Table 2]

|  | Mixing ratio of nanoparticles and liposomes ($X_{DM}$) |
|---|---|
| Com.Ex.1-1 | 0 |
| Com.Ex. 1-2 | 0.25 |
| Com.Ex. 1-3 | 0.5 |
| Com.Ex. 1-4 | 2 |

### Comparative Example 2. Preparation of dendritic cell-mimicked nanostructure without sonication

A dendritic cell-mimicked nanostructure was prepared by performing the same method as in Example 2, except for using a cell membrane suspension obtained from dendritic cells using a lysis buffer was used without sonication.

**Experimental Example 1. Evaluation of properties of dendritic cell-mimicked nanostructures according to Example 1 of the present invention and Comparative Example 1**

### 1-1. Surface coverage analysis

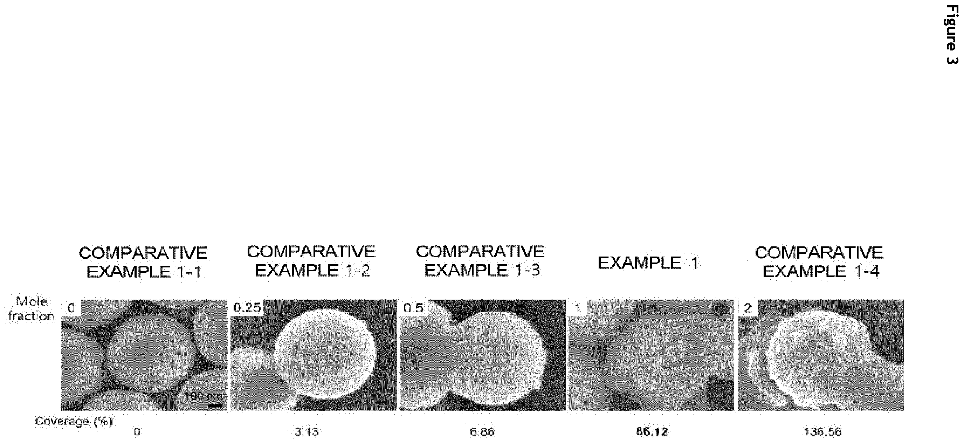

The surface coverage of the dendritic cell-mimicked nanostructures prepared according to Example 1 of the present invention and Comparative Example 1 was evaluated using a scanning electron microscope (7610F-plus, JEOL). The results were shown in FIG. 3. It may be seen that as the $X_{DM}$ value increases, the surface coverage also increases.

### 1-2. Surface zeta potential and PL intensity analysis

By using a dynamic light scattering nano particle size analyzer (DLS, SZ100, Horiba), the surface zeta potentials of the dendritic cell-mimicked nanostructures prepared according to Example 1 of the present invention and Comparative Example 1 were measured.
In addition, photoluminescence (PL) intensity was measured using a fluorescence spectrophotometer (FP-8300, JASCO).

### 1-3. T cell proliferation and differentiation induction assay

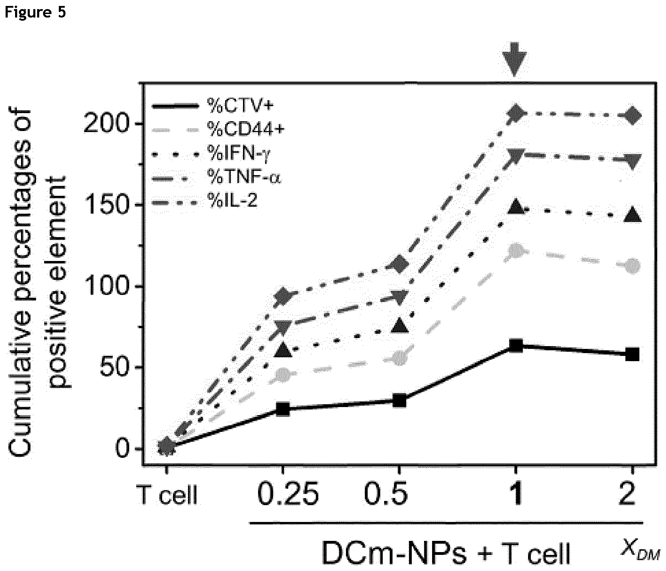

In a 96 well U bottom plate, dendritic cell-mimicked nanostructures (DCm-NP) prepared according to Example 1 and Comparative Example 1 using $2 \times 10^5$ CD8 T cells and $4 \times 10^4$ DC isolated from P14 mouse were added to the same well with 200 μl of 10% RPMI culture medium, and co-cultured at 37°C for 3 days. After 3 days, the plate was recovered from the incubator, stained with a fluorescent antibody, and the degree of activation of CD8 T cells was analyzed using flow cytometry. The results were shown in FIG. 5.
FIG. 5 is a graph of FACS data. In Example 1, where $X_{DM}$ was 1, when CTV was performed, CD44, IFNg, TNF$\alpha$, and IL-2 all showed the highest expression rates. It may be confirmed that the dendritic cell-mimicked nanostructure has excellent T cell proliferation and activation effects according to the optimized mixing ratio according to the present invention.

### 1-4. SDT data analysis

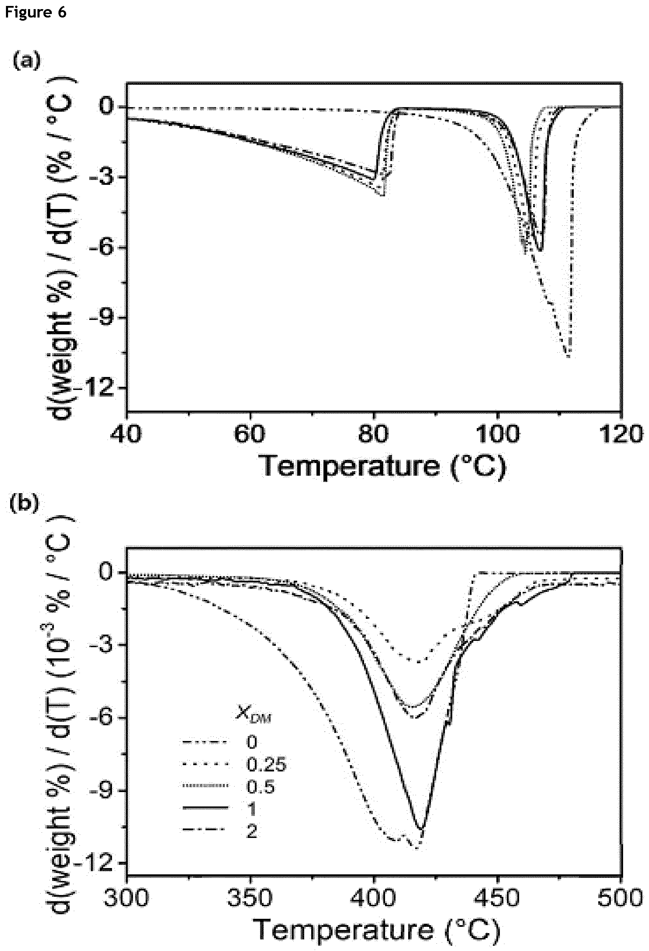

The thermal stability of each of $X_{DM}$ = 0, 0.25, 0.5, 1, and 2 was evaluated using a simultaneous DSC/TGA analyzer (SDT 650, TA instruments). A bilayer transition temperature ($T_m$), a glass transition temperature ($T_g$), and a melting temperature ($T_m$) were measured in an $N_2$ environment at a rate of 10°C/min from 30 to 1000°C, and the results were shown in Table 3 and FIG. 6 below.

[Table 3]

| $X_{DM}$ | $T_m$(°C) -bilayer transition temp. | $T_g$(°C) -Glass transition temp. | $T_m$(°C) -Melting temp. |
|---|---|---|---|
| | -Cell membrane | -Polystyrene (PS) | -Polystyrene (PS) |
| 0 | - | 111.52 | 417.29 |
| 0.25 | 80.98 | 105.37 | 416.38 |
| 0.5 | 81.53 | 104.44 | 415.50 |
| 1 | 79.83 | 106.99 | 418.95 |
| 2 | 82.50 | 106.86 | 416.86 |

FIG. 6A shows a graph for detailed analysis in the range of 40 to 150°C. In the case of $X_{DM}$ = 0, peaks at about 80°C shown at $X_{DM}$ = 0.25, 0.5, 1, 2 were not formed, and thus, peaks around 80°C appeared as peaks caused by the cell membrane.
Among $X_{DM}$ = 0.25, 0.5, 1, and 2 that showed peaks around 80°C, it may be seen that the peak of $X_{DM}$ = 1 was formed at the lowest temperature (79.83°C). As the number of double bonds increased, the bilayer transition temperature ($T_m$) decreased, and thus, this meant that in the case of $X_{DM}$ = 1, double bonds are most distributed in fatty acids constituting the membrane, and since almost 100% of the particle surface was covered, it was interpreted that the peak was formed at the lowest temperature due to the largest curvature. That is, it may be seen that when $X_{DM}$ = 1, the cell membrane is most stably formed on the particle surface.

Peaks were formed in all groups around 104°C to 112°C. Accordingly, the peaks were caused by polystyrene (PS) particles, and changes in the glass transition temperature ($T_g$) of PS may be confirmed. In the case of $X_{DM}$ = 0, a peak was formed at 111.52°C, which corresponded to the known glass transition temperature ($T_g$) range of PS. In the case of $X_{DM}$ = 0.25, 0.5, 1, 2, the $T_g$ values were lower than that of $X_{DM}$ = 0, which was because the cell membrane was coated. Considering that the peak was formed at the highest temperature (106.99°C) when $X_{DM}$ = 1, it may be confirmed that $X_{DM}$ = 1 was the most thermodynamically stable.

FIG. 6B shows a graph for detailed analysis in the range of 250 to 500°C. Peaks were formed at all of $X_{DM}$ = 0, 0.25, 0.5, 1, and 2, which were peaks caused by PS particles and indicated melting temperature ($T_m$). When comparing $X_{DM}$ = 0, 0.25, 0.5, 1, 2 respectively, when $X_{DM}$ = 1, the melting temperature ($T_m$) was formed at the highest temperature (418.95°C), which was interpreted as the most thermodynamically stable. This is a higher temperature than $X_{DM}$ = 0, which suggests that the structural stability of $X_{DM}$ = 1 is very high.

**Experimental Example 2. Evaluation of properties of dendritic cell-mimicked nanostructures according to Example 2 of the present invention and Comparative Example 2**

The surface zeta potentials of dendritic cell-mimicked nanostructures according to Example 2 of the present invention and Comparative Example 2 were analyzed.

[Table 4]

| | Zeta potential (mV) | |
|---|---|---|
| | Cell membrane suspension | Nanostructure |
| Ex. 1 (sonication) | -52.7 | -31.7 |
| Com. Ex. 2 (without sonication) | -61 | -60.65 |

As illustrated in FIG. 6, the nanostructure prepared according to Comparative Example 2, which was subjected to a co-extrusion process without sonication, showed almost no change in the value of surface zeta potential and a large variation. In particular, the variation of zeta potential was 20.7, which was a very large value. It was confirmed that after co-extrusion, a nanostructure coated with cell membrane-derived liposomes on gold nanoparticles and uncoated nanoparticles coexisted, and the cell membrane was not uniformly coated on the nanoparticles.

On the other hand, it may be seen that the absolute value of the surface zeta potential of the nanostructure prepared according to Example 2 sonicated is significantly reduced from 52.7 to 31.7. The cell membrane-derived liposomes also have a negative zeta potential due to a phospholipid structure, but the gold nanoparticles are dispersed in citric acid to have a stronger negative zeta potential. Since in the dendritic cell-mimicked nanostructure, the cell membrane was coated on the surfaces of gold nanoparticles with a strong negative zeta potential, as the cell membrane was well coated on the surface of the gold nanoparticles, the strong negative zeta potential of the gold nanoparticles was decreased. Thus, assuming that a relatively low negative zeta potential value will appear, it may be seen that the dendritic cell-mimicked nanostructure according to the present invention has a uniform cell membrane coating.

**Experimental Example 3. Analysis of properties of dendritic cell-mimicked nanostructure prepared according to Example 2**

Physicochemical and biological analyses were performed to confirm whether the dendritic cell-mimicked nanostructure according to Example 2 was properly prepared.

**3-1. TEM image analysis**

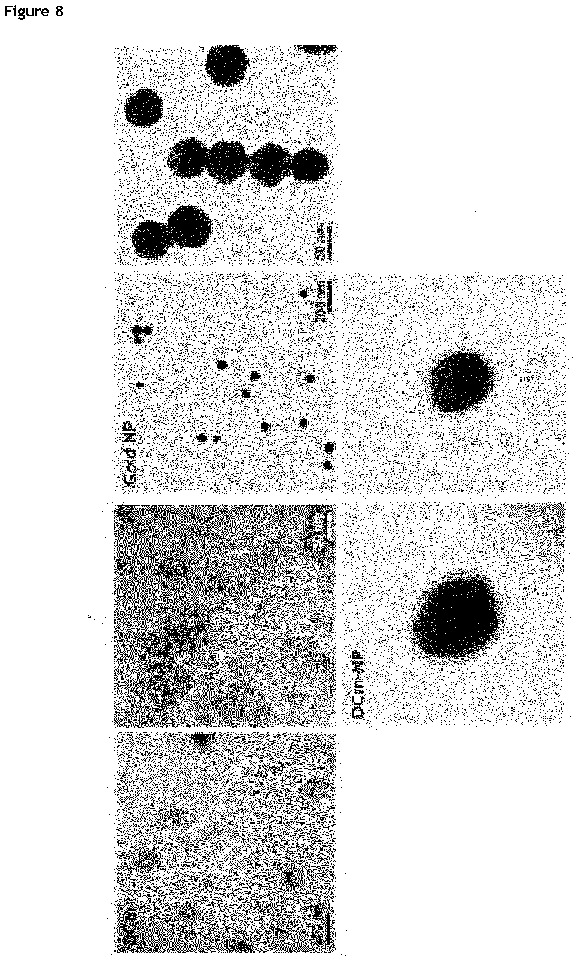

FIG. 8 illustrates a result of preparing a dendritic cell-mimicked nanostructure and analyzing the dendritic cell-mimicked nanostructure by TEM. It was confirmed that gold nanoparticles (gold NP) had high dispersion power and sizes of approximately 80 nm, and it was confirmed that although the extracted cell membrane (DCm) was not clear due to low electron density, liposomes of about 100 nm were formed. In the case of dendritic cell-mimicked nanostructure (DCm-NP), it was confirmed that the coating was formed with a thickness of approximately 10 to 20 nm, the dendritic cell-mimicked nanostructure was stably formed.

### 3-2. Zeta potential measurement

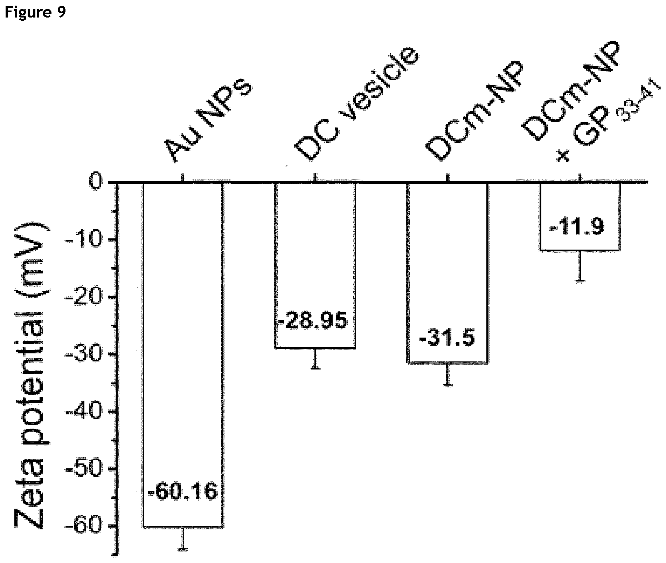

Through zeta potential measurement of gold nanoparticles (AuNPs), a dendritic cell membrane (DC vesicle), a dendritic cell-mimicked nanostructure (DCm-NP), and an antigen-pulsed dendritic cell-mimicked nanostructure (DCm-NP+GP$_{33-41}$), the surface charges were confirmed, and the results were illustrated in FIG. 9.

The gold nanoparticles had a strong negative zeta potential because the gold nanoparticles were dispersed in citric acid, and cell membrane liposomes also showed a negative zeta potential due to a phospholipid structure. It was confirmed that the dendritic cell-mimicked nanostructure had a cell membrane coated on the surface of gold nanoparticles with a strong negative zeta potential to have a relatively lower negative zeta potential value than gold nanoparticles. In addition, it may be confirmed that when an antigen GP-33 is loaded on the surface, the zeta potential value increases due to a weak positive charge within an amino acid sequence (Lys-Ala-Val-Tyr-Asn-Phe-Ala-Thr-Cys) of GP-33. As a result, it may be indirectly proven that the coated dendritic cell nanostructure was successfully prepared.

### 3-3. Confirmation of dendritic cell membrane protein distribution

Protein size distribution (SDS-PAGE) was confirmed to confirm whether immune-related proteins present in the dendritic cell membrane were not aggregated or denatured during the process of extracting the dendritic cell membrane, sonication, and filter extrusion.

The total protein distribution degree was confirmed through SDS-PAGE, in order to confirm protein denaturation that may occur during the process of preparing the dendritic cell-mimicked nanostructure and simultaneously confirm whether the cell membrane was stably coated on the surfaces of the nanoparticles. It was confirmed that there was no significant denaturation of the protein during the preparing step as there was no significant change in protein distribution before and after sonication during the intermediate step of preparing the nanostructure. The protein distribution of the finally prepared nanostructure also matched the protein distribution of the cell membrane, and as a result, it was confirmed that the cell membrane of the dendritic cell was successfully present in the nanostructure.

### 3-4. Analysis of dendritic cell membrane proteins

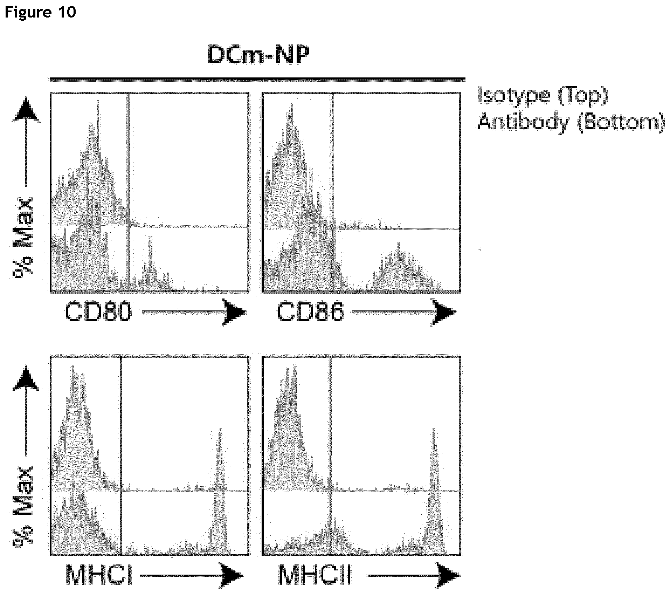

In order to confirm whether the cell membrane has been coated with gold nanoparticles in right-side-out membrane orientation during the filter extrusion process of gold nanoparticles and dendritic cell-derived cell membrane, the presence or absence of major immune-related membrane proteins, CD80, CD86, and MHC class I and II, in the prepared dendritic cell-mimicked nanostructure was confirmed through a flow cytometer (FACS). The results were illustrated in FIG. 10.

When the representative membrane proteins CD80, CD86, MHC class I, and II were analyzed using fluorescent antibodies, compared to a group that did not mimic dendritic cells, a positive fluorescence signal was confirmed when a dendritic cell-mimicked nanostructure (DCm-NP) was prepared and attached with an antibody, and as a result, it was confirmed that membrane proteins existed normally in the particle surface.

### 3-5. Induction of T cell proliferation and differentiation in vitro

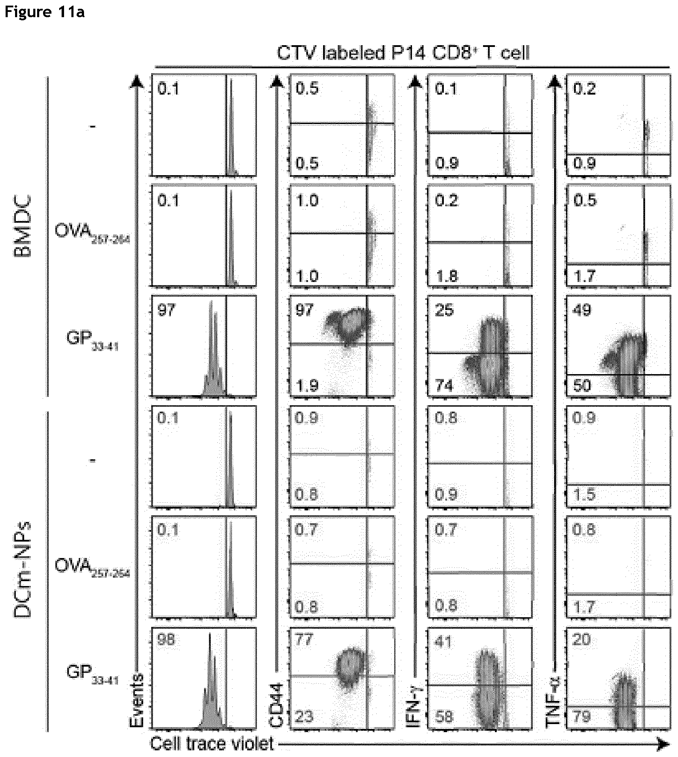

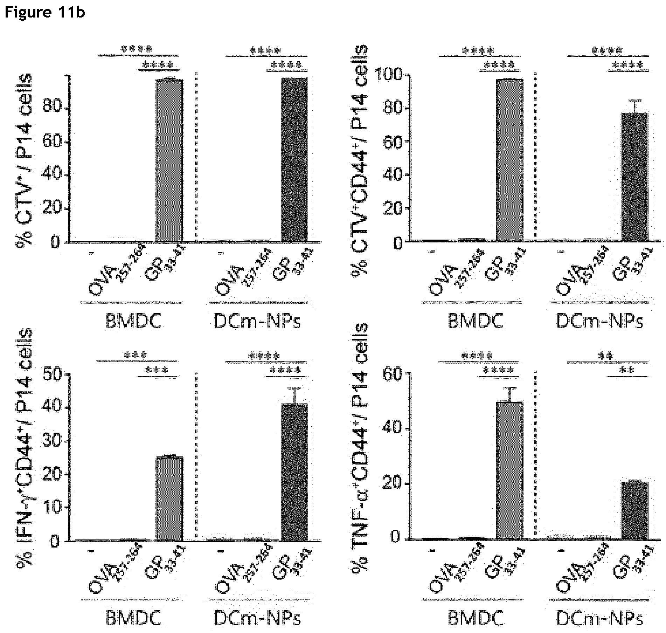

Co-culture analysis with T cells was performed to evaluate an effect of inducing T cell proliferation and differentiation of the dendritic cell-mimicked nanostructure in the same manner as Experimental Example 1-3, and the results were illustrated in FIGS. 11A and 11B.

When the dendritic cell-mimicked nanostructure was co-cultured with CD8 T cells without GP-33 treatment, it was confirmed that CTV, a measure of T cell proliferation, was not increased. Likewise, in the corresponding group, it was confirmed that CD44, an activation measure, and functional cytokines, such as IFNg, IL-2, and TNFa, were rarely produced from CD8 T cells. On the other hand, as the result of treating the dendritic cell-mimicked nanostructure with GP-33 to bind to the T cell receptor of CD8 T cells isolated from P14, it was confirmed that as CTV progressed, the CD8 T cells had proliferated, and it was confirmed that the CD8 T cells were activated through high CD44 expression. In addition, it was confirmed that IFNg and TNF$\alpha$ were significantly more released from the CD8 T cells. Through this, it was confirmed that even if only the cell membrane of the dendritic cell was isolated and mimicked to prepare a nanostructure, the nanostructure contributed to the proliferation and activation of antigen-specific CD8 T cells in vitro.

### 3-6. Induction of T cell proliferation and differentiation in vivo

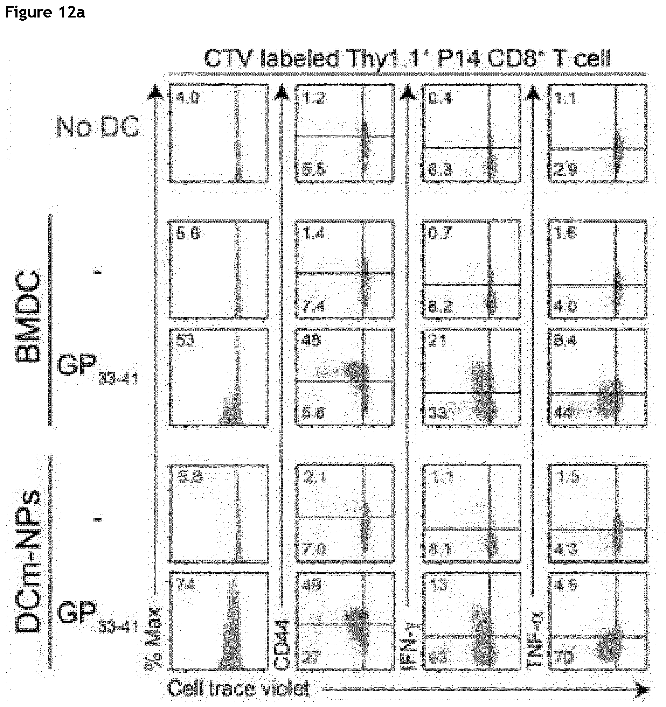

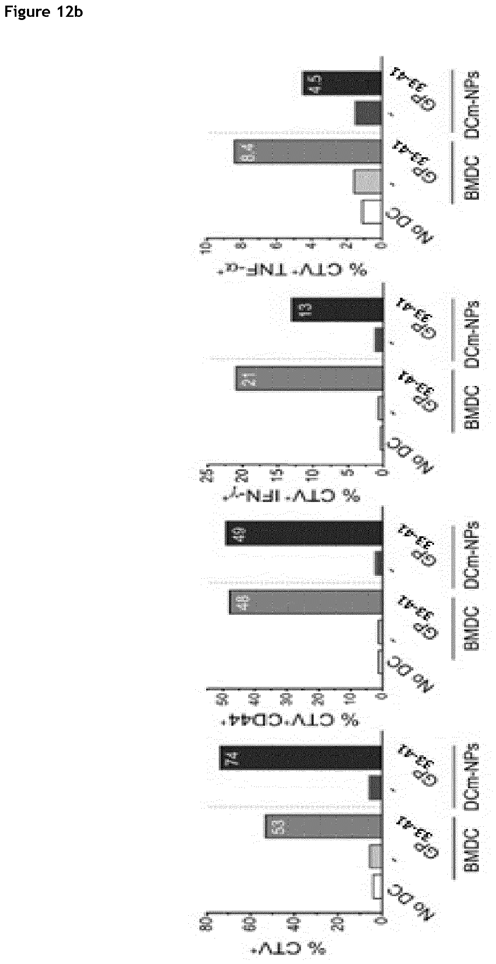

CD8 T cells were isolated from a P14 mouse with a congenic marker (Thy1.1) and then subjected to adoptive transfer to a naive recipient mouse, and after 24 hours, the adoptive transfer was performed with dendritic cells or dendritic cell-mimicked nanostructure under different conditions for each group. After 48 hours, immune cells were isolated from the spleen of the mouse, stained with a fluorescent antibody, and the activation degree of CD8 T cells was analyzed using a flow cytometer. The results were illustrated in FIGS. 12A and 12B.

G1 was a group added with only CD8 T cells, G2 was a group added with dendritic cells and CD8 T cells without treating GP$_{33-41}$, G3 was a group added with dendritic cells and CD8 T cells treated with GP$_{33-41}$, G4 was a group added with a dendritic cell-mimicked nanostructure and CD8 T cells without treating GP$_{33-41}$, and G5 was a group added with a dendritic cell-mimicked nanostructure and CD8 T cells treated with GP$_{33-41}$. In the case of G2 and G4 without treating GP-33, it was confirmed that all CD8 T cell activation indicators did not appear, like the negative control G1, whereas in G3, proliferation of CD8 T cells, activation through CD44, and secretion of functional cytokines IFNg and TNFα were confirmed through CTV. In the case of G5, it was confirmed that CD8 T cells were activated and proliferated to some extent compared to the negative control, although the degree was weaker than that of G3. As a result, it was confirmed that the dendritic cell-mimicked nanostructure treated with GP-33 may activate CD8 T cells in vivo.

### 3-7. Stability test

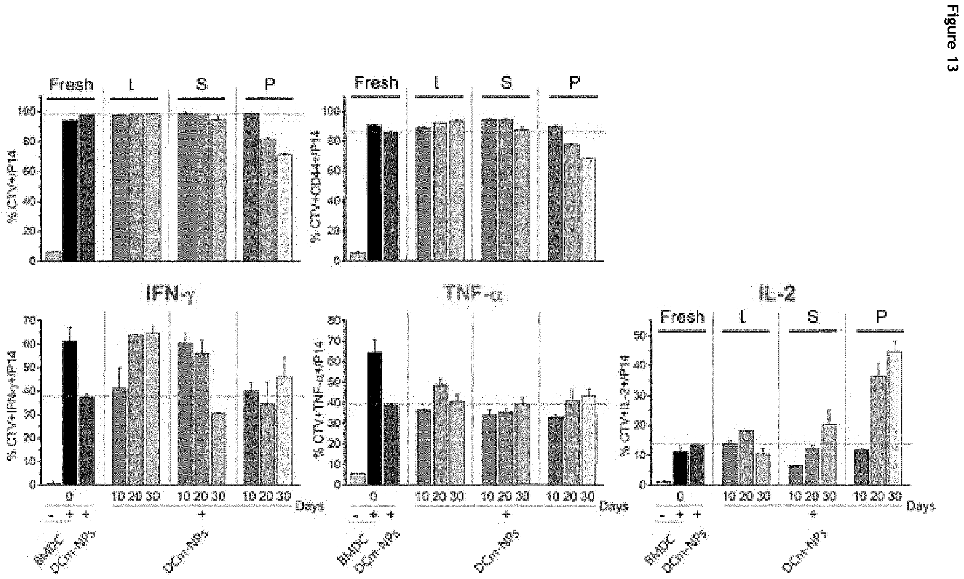

The dendritic cell-mimicked nanostructure (DCm-NP) prepared according to Example 2 was freeze-dried, stored at room temperature, dispersed in PBS, refrigerated (4°C), and frozen (- 20°C), and centrifuged. The pellets were refrigerated (4°C) and frozen (- 20°C), and the antigen was pulsed after 10, 20, and 30 days, respectively. T cell proliferation and differentiation efficiency was evaluated together with a group with the antigen pulsed to the dendritic cell-mimicked nanostructure (DCm-NP) and a group with/without pulsed antigen to bone marrow-derived dendritic cells. The results were illustrated in FIG. 13. It was confirmed that the dendritic cell-mimicked nanostructure (DCm-NP) according to the present invention maintained structural stability for 30 days or more and simultaneously also preserved the antigen presenting function of dendritic cells.

The present invention has been described with reference to Examples and Experimental Examples, but are merely illustrative examples, and various modified and equivalent other Examples and Experimental Examples are possible therefrom.

### Claims

1. A dendritic cell-mimicked nanostructure comprising a nanoparticle core; and a shell including a cell membrane of lipid molecules derived from dendritic cells, wherein the shell includes a double layer of lipid molecules.

2. The dendritic cell-mimicked nanostructure of claim 1, wherein the shell is 5 to 20 nm.

3. The dendritic cell-mimicked nanostructure of claim 1, wherein the surface coverage of the nanostructure is 70% or higher.

4. The dendritic cell-mimicked nanostructure of claim 1, wherein the surface coverage of the nanostructure is 85% or higher.

5. The dendritic cell-mimicked nanostructure of claim 1, wherein the nanostructure is fabricated by fusing liposomes prepared by sonicating the dendritic cell-derived cell membrane and the nanoparticles.

6. The dendritic cell-mimicked nanostructure of claim 5, wherein the absolute value of the surface potential of the nanostructure is smaller than the absolute value of the surface potential of liposomes of lipid molecules derived from dendritic cells.

7. The dendritic cell-mimicked nanostructure of claim 5, wherein the nanostructure is fabricated by co-extruding the liposomes and the nanoparticles.

8. The dendritic cell-mimicked nanostructure of claim 5, wherein the particle size of the liposome is 200 nm or less.

9. The dendritic cell-mimicked nanostructure of claim 1, wherein the surface zeta potential of the nanostructure is - 35 to - 25 mV.

10. A fabrication method of a dendritic cell-mimicked nanostructure comprising:

purifying a cell membrane from dendritic cells;

forming a cell membrane suspension by sonicating the cell membrane;
obtaining liposomes by filtering the cell membrane suspension through a membrane filter; and
mixing nanoparticles and the liposomes and then obtaining nanoparticles into which the cell membrane has been introduced by filter extrusion.

11. The fabrication method of the dendritic cell-mimicked nanostructure of claim 10, wherein the nanoparticles and the liposomes are mixed in a volume ratio of 1 : 150 to 1 : 300.

12. The fabrication method of the dendritic cell-mimicked nanostructure of claim 10, further comprising:
    pulsing an antigen to the nanoparticles into which the cell membrane has been introduced.

13. The fabrication method of the dendritic cell-mimicked nanostructure of claim 12, wherein the antigen is a peptide or protein derived from a tumor antigen.

14. The fabrication method of the dendritic cell-mimicked nanostructure of claim 10, wherein the average particle size of the liposome is 200 nm or less.

15. The fabrication method of the dendritic cell-mimicked nanostructure of claim 10, wherein the nanoparticles into which the cell membrane is introduced have a smaller absolute value of surface potential than the nanoparticles before the cell membrane introduction.

16. An immunotherapy comprising the dendritic cell-mimicked nanostructure according to any one of claims 1 to 9.

17. A pharmaceutical composition for treating cancer comprising the dendritic cell-mimicked nanostructure according to any one of claims 1 to 9.

18. A dendritic cell-mimicked nanostructure fabricated by the fabrication method of any one of claims 10 to 15.

**Figure 1**

**Figure 2**

**Figure 3**

**Figure 4**

**Figure 5**

Figure 6

(a)

(b)

**Figure 7**

Figure 8

Figure 9

Figure 10

DCm-NP

Isotype (Top)
Antibody (Bottom)

Figure 11a

**Figure 11b**

Figure 12a

Figure 12b

**Figure 13**

## INTERNATIONAL SEARCH REPORT

| International application No. |
| --- |
| **PCT/KR2021/013746** |

| A. | CLASSIFICATION OF SUBJECT MATTER |
| --- | --- |

**A61K 9/51**(2006.01)i; **A61K 39/00**(2006.01)i; **A61K 41/00**(2006.01)i; **A61P 35/00**(2006.01)i

According to International Patent Classification (IPC) or to both national classification and IPC

| B. | FIELDS SEARCHED |
| --- | --- |

Minimum documentation searched (classification system followed by classification symbols)

A61K 9/51(2006.01); A61K 39/00(2006.01); A61P 35/00(2006.01); C07K 14/47(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 수지상세포 (dendritic cell), 항원 제시 세포 (antigen presenting cells), T 세포 (T cell), 세포막 (cell membrane), 리포좀 (liposome), 금 나노입자 (gold nanoparticles), 초음파 (sonication), 압출 (extrusion)

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| X<br>Y | LIU, Wen-Long et al. Cytomembrane nanovaccines show therapeutic effects by mimicking tumor cells and antigen presenting cells. Nature Communications. 2019, vol. 10, article no. 3199, inner pp. 1-12, Supplementary information.<br>    See abstract, inner pages 1-10, figures 1-3; and supplementary figure 3. | 1-5,9,16,17<br>6-8,10-15,18 |
| Y | GAO, W. et al. Surface Functionalization of Gold Nanoparticles with Red Blood Cell Membranes. Advanced Materials. 2013, vol. 25, pp. 3549-3553.<br>    See pages 3549 and 3552; and figures 1 and 2A. | 6-8,10-15,18 |
| A | LUKASZ, J. et al. Dendritic Cell Membrane Vesicles for Activation and Maintenance of Antigen-Specific T Cells. Advanced Healthcare Materials. 2018, vol. 8, article no. 1801091, inner pp. 1-6.<br>    See abstract and figure 1. | 1-18 |
| A | CN 110090298 A (WUHAN UNIVERSITY) 06 August 2019 (2019-08-06)<br>    See abstract and claims 1-10. | 1-18 |

| ✓ Further documents are listed in the continuation of Box C. | ✓ See patent family annex. |
| --- | --- |

| * | Special categories of cited documents: |
| --- | --- |
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **24 January 2022** | **24 January 2022** |

| Name and mailing address of the ISA/KR | Authorized officer |
| --- | --- |
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

## INTERNATIONAL SEARCH REPORT

| International application No. |
| --- |
| **PCT/KR2021/013746** |

### C. DOCUMENTS CONSIDERED TO BE RELEVANT

| Category* | Citation of document, with indication, where appropriate, of the relevant passages | Relevant to claim No. |
| --- | --- | --- |
| A | GAO, W. et al. Coating nanoparticles with cell membranes for targeted drug delivery. Journal of Drug Targeting. 2015, vol. 23, nos. 7-8, pp. 619-626.<br>See abstract and pages 619-624. | 1-18 |
| A | KR 10-2003-0070454 A (CREAGENE INC.) 30 August 2003 (2003-08-30)<br>See entire document. | 1-18 |

INTERNATIONAL SEARCH REPORT
Information on patent family members

International application No.

**PCT/KR2021/013746**

| Patent document cited in search report | | | Publication date (day/month/year) | Patent family member(s) | | | Publication date (day/month/year) |
|---|---|---|---|---|---|---|---|
| CN | 110090298 | A | 06 August 2019 | None | | | |
| KR | 10-2003-0070454 | A | 30 August 2003 | KR | 10-0490308 | B1 | 17 May 2005 |

Form PCT/ISA/210 (patent family annex) (July 2019)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **HU ; CHE-MING J. et al.** *Nature,* 2015, vol. 526 (7571), 118-12
- **BRIAN T. LUK et al.** *Theranostics,* 2016, vol. 6 (7), 1004-101

- **Q. JIANG et al.** *Biomaterials,* 2019, vol. 192, 292-308